Europäisches Patentamt

(19) European Patent Office

Office européen des brevets

(11) Publication number: **0 018 179**
**B1**

(12) **EUROPEAN PATENT SPECIFICATION**

(45) Date of publication of patent specification: **30.05.84**

(21) Application number: **80301167.5**

(22) Date of filing: **11.04.80**

(51) Int. Cl.³: **A 61 M 25/00, A 61 M 31/00**

(54) Catheter tip for infusion.

(30) Priority: **13.04.79 US 29640**

(43) Date of publication of application:
**29.10.80 Bulletin 80/22**

(45) Publication of the grant of the patent:
**30.05.84 Bulletin 84/22**

(84) Designated Contracting States:
**AT BE CH DE FR GB IT LI LU NL SE**

(56) References cited:
**DE-B-1 491 793**
**FR-A- 900 765**
**US-A-3 592 184**
**US-A-3 658 053**
**US-A-3 995 617**
**US-A-4 029 104**

(73) Proprietor: **REGENTS OF THE UNIVERSITY OF MINNESOTA**
**100 Church Street Southeast**
**Minneapolis, Minnesota 55455 (US)**

(72) Inventor: **Dorman, Frank D.**
**3048 Hayes, N. E.**
**Minneapolis, Minnesota 55418 (US)**

(74) Representative: **Ellis, John Clifford Holgate et al**
**MEWBURN ELLIS & CO. 2/3 Cursitor Street**
**London EC4A 1BQ (GB)**

## Description

Technical Field

The present invention relates to a catheter tip for infusion.

Background of the Invention

This invention is directed to a catheter intended for implantation within a living body for long term operation. The catheter is intended for use, for example, with the implantable infusion pump or artificial gland described in United States Patent No. 3,731,681, or with other implanted injection ports for drug delivery to internal organs or sites not accessible by direct needle puncture.

The major problems with long term catheterization of a blood vessel are infection at the skin puncture point and clotting in the catheter lumen. A catheter of the type commonly employed is shown in French Patent 900,765. Only in cases where the flow rate of infusate is very high, or if an anticoagulent is being infused, does the catheter tip remain open for a long term. The catheter shown in U.S. Patent 3,995,617 is an example of a high pressure device although it is not an infusion type catheter. In applications such as insulin infusion, the flow rates are very low, and back diffusion of blood components can result in clots or plugs forming inside the lumen of the catheter. This limits the useful lifetime of the indwelling catheter in many instances to only three to four months.

In the case of use of an implanted infusion pump such as that of aforesaid United States Patent No. 3,731,681, a further complication exists in that the catheter does not always have a forward flow. For example, when the pump is being filled, it is possible to momentarily generate suction which pulls blood retrograde back into the catheter and this blood inevitably clots. Also, changes in blood pressure may cause a fluctuating pressure at the tip of the catheter and can induce backward flow up the catheter. The cyclic pressure pumps fluid along the wall where the velocity is low. The catheters are always designed with a small exit hole to keep the velocity of the infusate as high as possible. However, this also has danger in that the cross section is very small and can be easily plugged. The plug can come from material that diffuses upstream from the blood or from small particles which come downstream out of the pump itself. If the hole is made very small to obtain high velocity, it is difficult to keep it from plugging due to particulates in the infusate. The ideal catheter is one in which it is not necessary to maintain a fixed flow rate to keep it open. Therefore, the catheter flow rate may be allowed to go to zero for long periods of time. The catheter should also be able to pass particles that come downstream with the infusate without plugging the tip. In order to resist back diffusion, the exit hole should be as small as possible to maintain a critically high exit velocity. The catheter of the present invention incorporates these desirable features and overcomes the shortcomings of prior art devices.

Summary of the Invention

Broadly stated, the present invention is directed to a long term implantable infusion catheter having a check valve tip for unidirectional low level flow. The catheter is an elongated relatively thick walled tubular member having a central bore. The bore is open at the upstream end to receive infusate and is closed at the bottom end, as by a plug. At least one cross bore or port is provided in the wall of the tubular member communicating with the central bore and located upstream from and closely adjacent to the closed catheter end. A thin elastic sleeve having a diameter 5 to 30% less than that of the tubular member surrounds the outer surface of the tubular member in squeezing engagement. This sleeve covers the port. The sleeve is tightly secured against dislodgement from the tubular member, as by adhesive bonding or the like, upstream from the port. The remainder of the sleeve engages the outer wall of the tubular member in squeezing but unbonded relation. The pressure of infusate introduced through the catheter is sufficient to expand the elastic sleeve to permit fluid to pass from the port through the space created in the annular interface between the inside sleeve wall and outside catheter tip wall. When no infusate is applied the catheter is closed in both directions.

Brief Description of the Drawings

The invention is illustrated in the accompanying drawings in which corresponding parts are identified by the same numerals and in which:

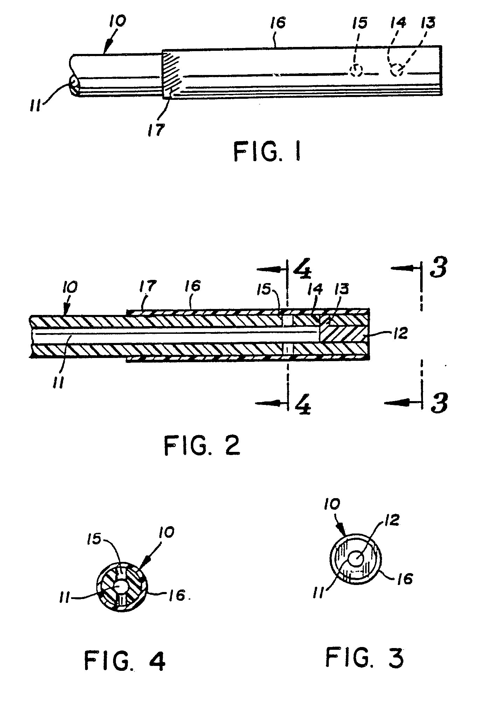

FIGURE 1 is a side elevation of one form of check valve catheter tip according to the present invention;

FIGURE 2 is a side elevation in section of the catheter tip shown rotated 90° from FIGURE 1;

FIGURE 3 is an end elevation of the catheter tip; and

FIGURE 4 is a transverse section through the ports taken on the line 4—4 of FIGURE 2 and in the direction of the arrows.

Detailed Description of the Invention

Referring now to the drawings, there is shown a relatively thick wall tubular catheter, indicated generally at 10, having a central bore 11 throughout its length. The catheter tube may be formed from any of a large number of flexible inert non-toxic bio-compatible rubber or synthetic rubber-like materials. A preferred material is a standard radio-opaque barium-filled silicone rubber which, apart from having excellent catheter properties, may be located using fluoroscopic techniques once implanted. Likewise, the catheter tube may have widely varying dimensions dependent upon the particular application to which it is to be put. One exemplary catheter tube is 0.236 cm (0.093 inch) outside diameter having a central bore of 0.041 cm (0.016 inch) diameter. It may be of whatever length is required to reach the desired infusion site.

The upstream end of bore 11 is open to receive fluid to be infused. The downstream end is closed, as by a short plug 12. As illustrated, plug 12 has a radial spur 13 extending into a transverse opening 14 in the wall of the catheter tip. While plug 12 may likewise be formed from any one of a number of biologically compatible materials, a preferred material is medical grade Silastic adhesive.

One convenient method of forming plug 12 is as follows: Hole 14 is bored through one wall of the catheter a short distance, such as 0.794 cm (5/16 inch) from the end. The catheter tube is clamped off upstream from hole 14 and a vacuum is applied to the open bore. Silastic adhesive paste is applied over hole 14 and drawn through the hole out through the end of the tube, thus forming plug 12. The excess paste is wiped from the outer surface and the plug is cured to insure a firm leak-proof bond between the plug and the wall of bore 11. The integrity of the plug seal is preferably tested by application of forward bias pressures of about 3.5 kg/sq. cm (50 psig) for 5 minutes.

The plugged catheter tip is then provided with a cross bore 15 to provide one or more ports from the catheter. Port 15 is located immediately upstream from plug 12, for example, about 0.476 cm (3/16 inch) in the exemplary catheter. The diameter of the exit port may be approximately the same diameter as the catheter bore.

A thin-walled elastic sleeve 16 is applied over the catheter tip so as to cover port 15. In the exemplary catheter, sleeve 16 may be about 3.175 cm (1.25 inches) long. Dependent upon the elasticity and strength of the material, it should be about 0.0013 to 0.025 cm (0.0005 to 0.01 inch) thick. The upstream end of sleeve 16 is sealed to the catheter tube by means of an adhesive bond extending around the periphery of the catheter tube.

Sleeve or sheath 16 is made undersized, approximately 5 to 30 percent, again dependent upon the elasticity and strength of the material, to generate a fixed compression at the end of the catheter. As an example, using the exemplary catheter having an outside diameter of 0.236 cm (0.093 inch), a sleeve of inside diameter of 0.190 cm (0.0748 inch) provides a squeeze factor of about 24.3 percent. That is, the outside catheter diameter (0.093 inch = 0.236 cm) divided by the inside sleeve diameter (0.0748 inch = 0.190 cm (equals 1.243. This value minus 1 and multiplied by 100 equals a 24.3 percent squeeze factor.

Sleeve 16 may likewise be formed from a number of different biologically compatible materials having the requisite elasticity. A preferred material is polyurethane polymer (Biomer). The forward opening pressure of the check valve catheter tip is a function of both the thickness of the sleeve and the squeeze factor, both of which may be controlled. The sleeve is desirably formed by dipping of the cylindrical mandrel of appropriate diameter into a solution of the polymer. The thickness of the resulting sleeve is directly proportional to the concentration of solvent in the polymer (viscosity), which rapidly changes with time as a function of exposure to air (humidity, temperature, etc.) and probably many other factors. Good results have been obtained using fresh unopened Biomer polyurethane in mixtures of 3 parts polymer to 1 part of N-N-dimethylacetamide thinner.

The urethane sleeve is desirably formed by first dipping the previously cleaned and dried mandrel into the polymer solvent and then quickly into the polymer solution itself. Once into the polymer solution, the solvent diffuses away from the mandrel surface carrying with it any air or other impurities, leaving a uniform coat. If the polymer solution contains entrained air, this is preferably purged from the solution by application of vacuum prior to use. The mandrel is desirably dipped into the solution with a slow twisting motion and then retracted with the same twisting motion. To form the exemplary sleeve which is 3.175 cm (1.25 inches) long, the mandrel is dipped into the solution about 3.8 cm (1.5 inches) and retracted at a rate of about 1.69 cm (2/3 inch) per minute. After the mandrel is free of the solution, it is rested in a vertical position on a drying stand. The force of gravity pulls off excess polymer, leaving a uniform coat varying from about 0.0038 cm (0.0015 inch) at the top to about 0.0051 cm (0.002 inch) at the bottom. The sleeve is then cured by heating at about 49°C (120°F) for about 36 hours.

Once cured, the sleeve is inspected for impurities, air bubbles, dust, etc., in the surface. It is trimmed to useful length and expanded off the mandrel by soaking in trichloroethylene for about 15 to 20 minutes. This expands the sleeve to about one and one-half ·times its normal diameter and about 10 percent in length. This permits easy transfer to the catheter tip. As the solvent evaporates, the cured sleeve returns to its original dimensions in tight squeezing engagement with the outer wall of the catheter tip. The sleeve is sealed to the wall of the catheter tip, as for example, by rolling the upstream end of the sleeve back a short distance, applying a biologically compatible adhesive, such as Silastic adhesive, to the catheter, quickly rolling the sleeve back over the adhesive and curing to form a permanent bond 17. The end of the catheter tip is preferably trimmed in a sharp cut.

Although polyurethane is a preferred material for formation of sleeve 16, other inert nontoxic rubber or synthetic rubber-like materials which are compatible with body fluids and which have the requisite elasticity can be used instead. When available, preformed tubular materials may be used to form the sleeve.

In use, the drug or other material to be infused is introduced into the end of the catheter under pressure, such as might be applied by the pump of the aforesaid U.S. Patent No. 3,731,681. The pressure of the fluid exiting from port 15 is sufficient to overcome the compressive force of sleeve 16 to cause the fluid to flow along the interface between the outside wall of the catheter tip and inside wall of the sleeve. The compressive force of the sleeve prevents back-flow into the catheter. Even where the infusate is introduced at a very low flow rate, such as insulin at the rate of approximately 1 cc per day, the flow rate at the exit slit at the end of the catheter tip at the interface between the sleeve and tip wall where the insulin enters the bloodstream is at a sufficiently high velocity to inhibit significant back-diffusion of blood components into the interface channel. Even if there is a small rate of buildup of deposit in this channel, the elastic nature of the sleeve is such that the sleeve may expand to permit continued flow with approximately the same pressure drop. The fluid channel at the interface may also move radially around the catheter until it finds a clean area which has been previously closed due to the elastic force and thus establish a new channel.

Ordinarily the flow rate is along one channel extending along the interface from the cross bore port to the free end of the catheter tip. Only when the fluid is introduced at very high flow rates will the sleeve expand to allow flow completely around the annular exit. This gives a very large effective exit cross section while at the same time maintaining a high exit velocity. Where the infusate may contain particles which might otherwise clog the catheter, the sheath either expands to allow the particle to pass through or traps the particle to allow flow around it. The catheter tip is thus unpluggable from both directions due to the expandability of the sleeve. Negative pressures as great as —1.4 kg/sq. cm (—20 psi) ($P_{in} - P_{out}$) have been witnessed without failure. These are pressures far beyond those that would be expected in clinical situations.

Although intended primarily for use with an implantable pump or artificial gland, such as described in aforesaid U.S. Patent No. 3,731,681, the catheter may also be used with only an implanted septum. For example, a catheter is surgically implanted in a difficult-to-place location, such as a vein or artery going into some internal organ that is not accessible by needle puncture from the surface of the body. After this catheter has been implanted and a puncture septum is placed at a convenient location under the skin, the internal organ can be perfused with any infusate by simple puncture of the readily accessible external puncture point. The use of this technique avoids the necessity for passing the catheter through the skin and the chronic problems associated therewith.

**Claims**

1. A catheter tip for infusion comprising:
an elongated relatively thick-walled tubular member,
a central bore in said member, said bore being open at the upstream end and closed at the downstream end,
at least one port in the wall of the tubular member communicating with said bore, said port being located upstream from said closed end of the tubular member and closely adjacent thereto,
characterized by the provision of a check valve for unidirectional low level flow out of the catheter, said check valve comprising a thin elastic sleeve surrounding and normally biased against the outer surface of said tubular member in squeezing engagement therewith, said sleeve having a diameter 5—30% less than the outside diameter of said tubular member and covering said port, such that said sleeve seals said port from flows into said catheter at all times and from all flows when no fluid pressure is applied from within said catheter,
means securing said sleeve to the tubular member upstream from the port.

2. A catheter tip according to claim 1 wherein the closed end of said bore includes a plug in fluid-tight sealing engagement with the bore wall.

3. A catheter tip according to claim 2 wherein said tubular member has a radial hole communicating with the bore and said plug has a radial spur engaging said hole in fluid-tight sealing engagement.

4. A catheter tip according to any one of the preceding claims wherein said thin elastic sleeve is between about 0.0013 and 0.025 cm (0.005 and 0.01 inch) in thickness.

5. A catheter tip according to any one of the preceding claims wherein said means for securing the sleeve to the tubular member is an adhesive bond between the inside wall of the sleeve and the outside wall of the tubular member.

6. A catheter tip according to any one of the preceding claims wherein said tubular member is composed of silicone rubber and said sleeve is composed of polyurethane.

7. A catheter tip according to claim 6 wherein said silicone tubular member is barium-filled and radio-opaque.

8. A catheter tip according to any one of the preceding claims wherein the closed end of the catheter tip and the end of the sleeve terminate in a plane transverse to the longitudinal axis of the catheter to provide a high velocity annular jet port.

## Revendications

1. Embout de cathéter pour perfusion comprenant:

un élément tubulaire allongé à paroi relativement épaisse.

une lumière centrale située dans ledit élément, ladite lumière étant ouverte à l'extrémité amont et fermée à l'extrémité aval,

au moins un orifice ménagé dans la paroi de l'élément tubulaire et communiquant avec ladite lumière, ledit orifice étant situé en amont de ladite extrémité fermée de l'élément tubulaire et étroitement adjacent à cette extrémité,

caractérisé par la présence d'un clapet de retenue pour un écoulement de sortie unidirectionnel de faible niveau, du cathéter, ledit clapet de retenue comprenant un manchon élastique mince entourant la surface extérieure de l'élément tubulaire contre laquelle il est normalement rappelé élastiquement en contact serré, ledit manchon ayant un diamètre inférieur de 5—30 % diamètre extérieur dudit élément tubulaire et recouvrant ledit orifice, de manière que ledit manchon obture ledit orifice pour empêcher constamment des écoulements vers l'intérieur dudit cathéter et tous écoulements lorsque'aucune pression de fluide n'est appliquée de l'entérieur dudit cathéter,

des moyens fixant ledit manchon à l'élément tubulaire en amont de l'orifice.

2. Embout de cathéter selon la revendication 1, dans lequel l'extrémité fermée de ladite lumière comporte un bouchon en contact d'obturation étanche aux fluides avec la paroi de la lumière.

3. Embout de cathéter selon la revendication 2, dans lequel ledit élément tubulaire présente un trou radial communiquant avec la lumière et ledit bouchon comporte un ergot radial s'engageant dans ledit trou pour l'obturer de manière étanche aux fluides.

4. Embout de cathéter selon l'une quelconque des revendications, précédentes, dans lequel ledit manchon élastique mince présente une épaisseur comprise entre environ 0,0013 et 0,025 cm (0,0005 et 0,01 inch).

5. Embout de cathéter selon l'une quelconque des revendications précédentes, dans lequel lesdits moyens pour fixer le manchon à l'élément tubulaire comprennent une liaison adhésive entre la paroi inférieure du manchon et la paroi extérieure de l'élément tubulaire.

6. Embout de cathéter selon l'une quelconque des revendications précédentes, dans lequel ledit élément tubulaire est composé d'un caoutchouc siliconé et ledit manchon est composé d'un polyuréthanne.

7. Embout de cathéter selon la revendication 6, dans lequel ledit élément tubulaire siliconé est chargé de baryum et radio-opaque.

8. Embout de cathéter selon l'une quelconque des revendications précédentes, dans lequel l'extrémité fermée de l'embout de cathéter et l'extrémité du manchon aboutissent dans un plan transversal à l'axe longitudinal du cathéter pour former un orifice à jet annulaire à grande vitesse.

## Patentansprüche

1. Katheterspitze für Infusionen mit einem langgestreckten, verhältnismäßig dickwandigen röhrenförmigen Element, einer zentralen Bohrung in diesem Element, die am stromaufwärts gelegenen Ende offen und am stromabwärts gelegenen Ende geschlossen ist, mit wenigstens einer, in der Wand des röhrenförmigen Elementes vorgesehenen Öffnung, die mit der Bohrung kommuniziert, wobei die Öffnung stromaufwärts vom geschlossenen Ende des röhrenförmigen Elements und nahe an diesem angeordnet ist, dadurch gekennzeichnet, daß ein Absperr- bzw. Rückschlagventil für den in einer Richtung fließenden geringen Strom aus dem Katheter vorgesehen ist, welches Absperrventil eine dünne, elastische Hülse umfaßt, welche die außenfläche des röhrenförmigen Elementes umgibt, gegen diese normalerweise vorgespannt ist und sich in Preßberührung mit dieser befindet, daß die Hülse einen Durchmesser besitzt, der um 5—30% geringer ist als der Außendurchmesser des röhrenförmigen Elementes und die Öffnung überdeckt, so daß die Hülse die Öffnung jederzeit gegen Einströmen in den Katheter und gegen jedes Strömen abdichtet, wenn kein Fluiddruck von innerhalb des Katheters anliegt, und daß eine Einrichtung für die Befestigung der Hülse am röhrenförmigen Element stromaufwärts von der Öffnung vorgesehen ist.

2. Katheterspitze nach Anspruch 1, dadurch gekennzeichnet, daß das geschlossene Ende der Bohrung mit einem Pfropfen bzw. Verschluß versehen ist, der in fluiddichtem Dichtungseingriff mit der Bohrungswand steht.

3. Katheterspitze nach Anspruch 2, dadurch gekennziechnet, daß das röhrenförmige Element ein radiales Loch aufweist, das mit der Bohrung kummuniziert und daß der Pfropfen bzw. Verschluß mit einem radialen Sporn bzw. Ausläufer versehen ist, der mit dem Loch in fluiddichtem Dichtungseingriff steht.

4. Katheterspitze nach einem der vorhergehenden Ansprüche, dadurch gekennzeichnet, daß die Dicke der dünnen elastischen Hülse etwa 0,0013—0.025 cm (0,0005—0,01 inch) beträgt.

5. Katheterspitze nach einem der vorhergehenden Ansprüche, dadurch gekennziechnet, daß die Einrichtung für die Befestigung der Hülse am röhrenförmigen Element eine Klebebindung zwischen der Innenwand der Hülse und der Außenwand des röhrenförmigen Elementes ist.

6. Katheterspitze nach einem der vorhergehenden Ansprüche, dadurch gekennzeichnet, daß das röhrenförmige Element aus Silikongummi und die Hülse aus Polyurethan besteht.

7. Katheterspitze nach Anspruch 6, dadurch gekennzeichnet, daß das aus Silikon bestehende röhrenförmige Element mit Barium gefüllt
und strahlenunurchlässig ist.

8. Katheterspitze nach einem der vorhergehenden Ansprüche, dadurch gekennzeichnet,
daß das geschlossene Ende der Katheterspitze
und das Ende der Hülse in eine Ebene münden,
die in Querrichtung in bezug auf die Längsachse
des Katheters verläuft, um eine ringförmige
Düsenöffnung für hohe Strömungsgeschwin-
digkeiten zu schaffen.

FIG. 1

FIG. 2

FIG. 4

FIG. 3